(19) Europäisches Patentamt
European Patent Office
Office européen des brevets

(11) Veröffentlichungsnummer: **0 318 795**
**A2**

# (12) EUROPÄISCHE PATENTANMELDUNG

(21) Anmeldenummer: **88119382.5**

(51) Int. Cl.4: **C07D 475/08 , A61K 31/52**

(22) Anmeldetag: **22.11.88**

(30) Priorität: **28.11.87 DE 3740441**

(43) Veröffentlichungstag der Anmeldung: **07.06.89 Patentblatt 89/23**

(84) Benannte Vertragsstaaten: **AT BE CH DE ES FR GB IT LI NL SE**

(71) Anmelder: **Röhm GmbH**
**Kirschenallee Postfach 4242**
**D-6100 Darmstadt 1(DE)**

(72) Erfinder: **Hofmann, Ingrid, Dr.**
**Merzigerweg 1A**
**D-6000 Frankfurt/M. 71(DE)**
Erfinder: **Mutschler, Ernst, Prof. Dr.**
**Am Hechenberg 24**
**D-6500 Mainz-Hechtsheim(DE)**
Erfinder: **Christner, Angelika, Dr.**
**Bahnhofstrasse 42**
**D-6101 Bickenbach(DE)**

## (54) Pharmazeutisch wirksame Pteridinderivate.

(57) Die Erfindung betrifft Pharmazeutisch wirksame Pteridin-Verbindungen der Formel I

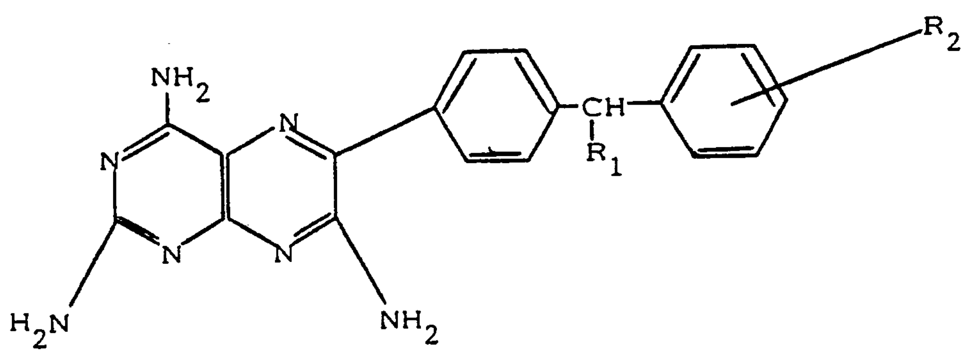

I

worin $R_1$ für Wasserstoff, für ein Halogen, für eine Trifluormethylgruppe, für eine Nitrogruppe, für eine $-(A)_n-OR_3$-Gruppe, wobei $R_3$ Wasserstoff oder einen Alkylrest mit 1 bis 6 Kohlenstoffatomen bedeutet, für eine $-(A)_n-NR_4R_5$-Gruppe, worin $R_4$ und $R_5$ unabhängig voneinander Wasserstoff oder einen Alkylrest mit 1 bis 6 Kohlenstoffatomen bedeuten oder $R_4$ oder $R_5$ zusammen mit dem Stickstoff einen 5- oder 6-gliedrigen, gegebenenfalls mit einer Alkylgruppe mit 1 bis 4 Kohlenstoffatomen substituierten Ring bilden, steht und worin A für eine abstandhaltende Alkylengruppe mit 1 bis 6 Kettengliedern, wovon eines durch Sauerstoff ersetzt sein kann und n für null oder eins steht und
worin $R_2$ für Wasserstoff steht oder - und unabhängig von $R_1$ - die gleichen Bedeutungen wir $R_1$ besitzt, mit der Maßgabe, daß $R_1$ und $R_2$ nicht gleichzeitig für Wasserstoff stehen, und die davon abgeleiteten, physiologisch tolerierbaren Säureadditionssalze.

EP 0 318 795 A2

## Gebiet der Erfindung

Die Erfindung betrifft pharmazeutisch wirksame, insbesondere antiarrhythmisch und diuretisch wirksame Pteridinderivate.

Stand der Technik

Im Jahre 1944 hatten Lippschitz und Hadidian (W.L. Lippschitz, Z. Hadidian, J. Pharmacol. Exp. Ther. 81, 84 -94 (1944); E. Mutschler & H. Knauf Hrsg., 30 Jahre Triamteren, Wissenschaftsverlag Köln, 1984, S. 11 - 18) eine Hypothese über den Zusammenhang von chemischer Struktur und diuretischer Wirksamkeit stickstoffhaltiger Verbindungen formuliert, derzufolge das Struckturelemente - N - C - N -eine Voraussetzung für die diuretische Aktivität darstelle. Obwohl sachlich nicht haltbar, führte diese Hypothese dennoch zur Entwicklung einer Reihe von diuretisch wirksamen Strukturen aus der Klasse der Purine, Pyrimidine, Triazine, des Uracils, der Pteridine und weiterer Heterocyclen, darunter das 2,4,7-Triamino-6-phenylpteridin (US-A 3 081 230) das sich als ein hervorragendes Anti-Kaliuretikum erwiesen hat (Triamteren). Der Zusammenhang zwischen chemischer Struktur und diuretischer Wirkung wurde von Weinstock & Wiebelhaus an ca. 500 Pteridinderivaten untersucht (vgl. K. Fellinger, Therapie mit Triamteren, Georg Thieme Verlag 1967, S. 2 - 21), die getroffenen Korrelationen sind jedoch nicht unwidersprochen geblieben. (Vgl. E. Wolf in E. Mutschler & H. Knauf, 30 Jahre Triamteren, loc.cit. S. 11 - 18).
G.H. Mudge stellt daher in Goodman-Gilman "The Pharmacological Basis of Therapeutics", 5th Ed. Mc. Millan Publishing Co., Inc. S. 838 zu Triamteren fest: "It is a pteridine compound, related chemically to folic acid. The diuretic activity of closely related homologs of triamterene has been examined, but no specific structural requirements have been established."
Entgegen den von Weinstock & Wiebelhaus entwickelten Vorstellungen können Derivate des Triamterens, bei denen die para-Stellung des 6-Phenylrings mit einer hydrophilen Gruppe veräthert ist, diuretische und antikaliuretische Wirkung besitzen (GB-C 1 597 881; DE-A 34 07 695). Aus der US-A 4 621 085 (bzw. DE-A 34 12 765) sind herzwirksame insbesondere antiarrhythmisch wirksame pharmazeutische Mittel bekannt, die als Wirkstoff ein Triamterenderivat enthalten, dessen 6-Phenylrest in para-Stellung lipophil substituiert ist. Als solche lipophile Substituenten werden Fluor, Chlor, verzweigtes oder cyclisches Alkyl mit 3 bis 6 Kohlenstoffatomen, Benzyl, Trifluormethyl oder Nitrosubstituenten spezifisch genannt.

Aufgabe und Lösung

Durch die in der jüngeren Patent-Literatur zur Verfügung gestellten Triamterenderivate wurden die früheren Konzepte über Struktur-Wirkungsbeziehungen sehr in Frage gestellt. Offenbar bestehen recht komplexe Zusammenhänge, so daß die einschlägige Feststellung in der DE-A 34 07 697 ihre Bereichtigung behält: "In das Profil eines Wirkstoffs ... gehen indessen zahlreiche Parameter ein, wie z.B. Wirksamkeit, akute und chronische Toxizität, Kompatibilität mit anderen Wirkstoffen, Pharmakokinetik und -dynamik, Metabolismus, physikalische und chemische Parameter wie Stabilität, Löslichkeit, Zugänglichkeit u.a." Der Stand der Technik bot keine Kriterien, in welcher Richtung eine Weiterentwicklung bestehender Strukturen in Richtung verbesserter Eigenschaften zu suchen wäre, an. Insbesondere bestand ein Bedarf an oral applizierbaren Verbindungen, wobei jedoch an die übrigen pharmakologischen Eigenschaften keine geringeren Anforderungen zu richten waren als an den Stand der Technik. Es wurde gefunden, daß Derivate des para-Benzyltriamterens diesen Forderungen überraschenderweise sehr nahe kommen bzw. sie sogar übertreffen.
Die erfindungsgemäßen Wirkstoffe werden durch die Formel I

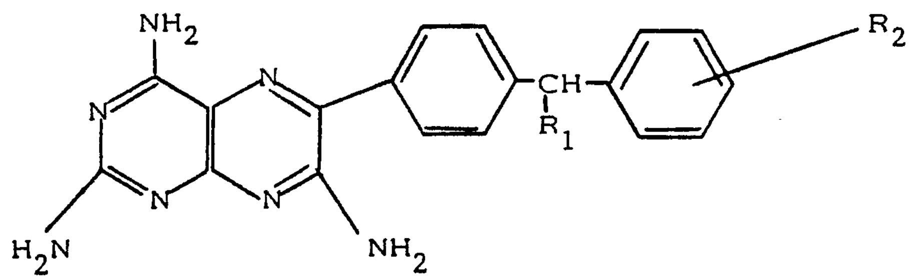

I

worin R1 für Wasserstoff, für ein Halogen, für eine Trifluormethylgruppe für eine $-(A)_n-OR_3$-Gruppe, worin $R_3$ Wasserstoff oder einen Alkylrest mit 1 bis 6, vorzugsweise 1 bis 3, insbesondere 1 bis 2 Kohlenstoffatomen bedeutet, für eine $-(A)_n-NR_4R_5$-Gruppe, worin $R_4$ und $R_5$ unabhängig voneinander Wasserstoff oder einen Alkylrest mit 1 bis 6, vorzugsweise 1 bis 2, Kohlenstoffatomen bedeuten
oder $R_4$ und $R_5$ zusammen mit dem Stickstoff und gegebenenfalls unter Einbeziehung eines weiteren Stickstoff- oder Sauerstoffatoms einen 5- oder 6-gliedrigen, gegebenenfalls mit einer Alkylgruppe mit 1 bis 2 Kohlenstoffatomen substituierten Ring bilden, oder $R_1$ für eine Nitrogruppe steht und worin A für eine abstandhaltende Gruppe, vorzugsweise eine Alkylenkette mit 1 bis 6 Kettengliedern und worin n für null oder eins steht, und
worin $R_2$ für Wasserstoff steht oder die gleichen Bedeutung wie $R_1$ besitzt, mit der Maßgabe, daß $R_1$ und $R_2$ nicht gleichzeitig für Wasserstoff stehen sollen und die davon abgeleiteten, physiologisch tolerierbaren Säureadditionssalze, wiedergegeben.

Vorzugsweise steht in den erfindungsgemäßen Verbindungen jedoch eine der beiden Gruppen, $R_1$ oder $R_2$ für Wasserstoff. Genannt seien Verbindungen, worin $R_1$ bzw. $R_2$ für ein Halogen mit einem Atomgewicht von 19 bis 80 steht. Bevorzugt sind Verbindungen, worin $R_1$ für -OH steht.
Sofern $R_2$ von Wasserstoff verschieden ist, steht dieser Substituent vorzugsweise in para-Stellung zum benzylischen C-Atom. Somit stellt der Substituent in para-Stellung des para-Benzyltriamterens vorzugsweise eine Fluor-, Chlor-, Brom-, Trifluomethyl-, Hydroxy-, gegebenenfalls substituierte Amino- oder Nitrogruppe dar.
Ferner sind bevorzugte Vertreter der erfindungsgemäßen Verbindungsklasse, die eine unsubstituierte Phenylgruppe bei Hydroxy-, gegebenenfalls substituierter Amino- oder Nitro-Substitution am benzylischen C-Atom aufweisen. Soweit $-NR_4R_5$ zusammen mit dem Stickstoffatom und gegebenenfalls unter Einbeziehung eines weiteren Stickstoff- oder Sauerstoffatoms einen 5- oder 6-gliedrigen, gegebenenfalls mit einer $C_1$-$C_2$-Alkylgruppe substituierten Ring bildet, handelt es sich vorzugsweise um einen Morpholinyl-, Piperidinyl-, Pyrrolidinyl- oder gegebenenfalls alkyl-, insbesondere $C_1$-$C_4$-alkyl-substituierten Piperazinylrest. Die Verbindungen der Formel I besitzen ein chirales Zentrum. Die vorliegenden Ansprüche sollen alle hinsichtlich der Chiralität unterscheidbaren Formen umfassen. Die Verbindungen sind im allgemeinen gelbliche, kristalline, hochschmelzende Körper. Die Verbindungen der Formel I zeigen im UV-Licht die den Pteridinverbindungen eigene starke Fluoreszenz, die z.B. auch beim Nachweis ausgenützt werden kann.
Genannt seien die folgenden Verbindungen der Formel I:
2,4,7-Triamino-6-[4-(4′-chlorbenzyl)-phenyl]-pteridin (p-Chlorbenzyl-Triamteren)
2,4,7-Triamino-6-[4-(4′-fluorbenzyl)-phenyl]-pteridin (p-Fluorbenzyl-Triamteren)
2,4,7-Triamino-6-[4-(4′-brombenzyl)-phenyl]-pteridin (p-Brombenzyl-Triamteren)
2,4,7-Triamino-6-[4-(4′-trifluormethylbenzyl)-phenyl]-pteridin(p-Trifluormethyl-benzyltriamteren)
2,4,7-Triamino-6-[4-(4′-hydroxybenzyl)-phenyl]-pteridin (p-Hydroxybenzyl-Triamteren)
2,4,7-Triamino-6-[4-(4′-nitrobenzyl)-phenyl]-pteridin (p-Nitrobenzyl-Triamteren)
2,4,7-Triamino-6-[4-(4′-aminobenzyl)phenyl]-pteridin (p-aminobenzyl-Triamteren)
(Phenyl-)[4-(2,4,7-Triamino-pteridinyl-6)-phenyl]-methanol [p-(α-Hydroxybenzyl)-triamteren] und das davon abgeleitete p-Nitrophenyl-, p-Chlorphenyl-, p-Fluorophenyl-, p-Bromphenyl-p-Hydroxyphenyl-, p-Aminophenyl-, p-Dimethylaminophenyl-derivat und die davon abgeleiteten Ether, insbesondere der Methyl- und der Ethylether, α-(4-(2,4,7-Triamino-pteridinyl-16)-phenyl]benzyldimethylamin (p-Benzyl-dimethylamino-Triamteren) und die am Benzylrest insbesondere in para-Stellung mit Fluor-, Chlor-, Brom-, Amino-, Nitro- oder Hydroxygruppen substituierten Derivate, (Phenyl-) [4-(2,4,7-Triamino-pteridinyl-6)-phenyl]nitromethan und die im Phenylrest, insbesondere in para-Stellung mit Fluor-, Chlor-, Brom-, Amino-, Nitro- oder Hydroxygruppen substituierten Derivate.

Die Herstellung der erfindungsgemäßen Verbindungen der Formel I kann gemäß dem folgenden Syntheseschema bzw. in Anlehnung an dasselbe erfolgen (wobei jedoch andere Synthesemöglichkeiten nicht ausgeschlossen werden sollen): Die Herstellung der Endprodukte der Formel I kann prinzipiell in

Anlehnung an die Triamterensynthese von R.G. W. Spickett & G.M. Timmis in J.Chem. Soc. 2887 (1954) durch Ringschlußreaktion eines substituierten Benzylcyanids der Formel II

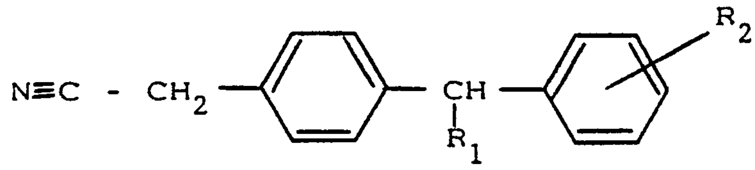

II

worin $R_1$ und $R_2$ die oben bezeichneten Bedeutungen besitzen, mit 2,4,6-Triamino-5-nitrosopyrimidin der Formel III

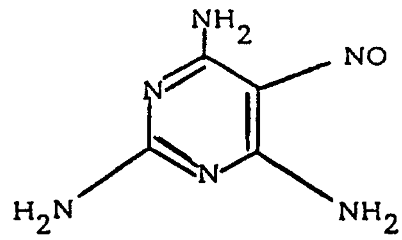

III

unter zur Kondensation geeigneten Bedingungen erfolgen. Diese Bedingungen umfassen z.B. die Umsetzung der Verbindung II mit III unter Basenkatalyse und in einem geeigneten Lösungsmittel, beispielsweise einem Alkohol insbesondere einem Ether-Alkohol wie 1-Methoxy-2-propanol. Als Base kann z.B. das durch Lösen eines Alkalimetalls, insbesondere des Natriums in dem Alkohol erzeugte Alkalialkoholat Verwendung finden. Es kann nützlich sein, die Verbindung der Formel II in einem gewissen, meist nur geringfügigen Überschuß gegenüber der Verbindung der Formel III anzuwenden.
Vorzugsweise erfolgt die Umsetzung unter Erwärmen beispielsweise bei ca. 70 - 120 Grad C, vorteilhafterweise bis zum Siedepunkt des Alkohols, d.h. am Rückfluß. Im allgemeinen genügt eine Reaktionsdauer von einigen Stunden; als Anhaltspunkt seien 2 Stunden genannt.
Zur Aufarbeitung wird zweckmäßig auf Raumtemperatur abgekühlt und vorteilhafterweise dann durch Zusatz eines Lösungsmittels die Fällung ausgelöst, die durch Stehenlassen beispielsweise bei Temperaturen unterhalb Raumtemperatur vervollständigt werden kann.

Die Reinigung der Verbindung der Formel I kann in an sich bekannter Weise, beispielsweise durch Überführung in ein Säureadditionssalz, beispielsweise mittels wäßriger Essigsäure, Lösen in der Siedehitze, gegebenenfalls unter Zusatz von Aktivkohle und Abkühlenlassen unter Kristallisation erfolgen.
Aus der Säureadditionsverbindung kann durch Behandlung mit einer geeigneten, vorzugsweise wäßrigen Base, z.B mittels konz. Ammoniaklösung die Verbindung der Formel I freigesetzt werden.
Die Ausbeuten sind unterschiedlich, sie können aber > 90 % d. Th. liegen.

Die Verbindungen der Formel I fallen in der Regel kristallin an. Sie können beispielsweise dünnschichtchromatographisch auf Reinheit geprüft werden.
NMR-Daten und Elementaranalyse bestätigen die angegebenen Strukturen.

Die Herstellung der Verbindungen der Formel II erfolgt nach an sich bekannten Methoden bzw. in Anlehnung an an sich bekannte Methoden. Als eine Schlüsselverbindung kann beispielsweise eine Verbindung der Formel IV

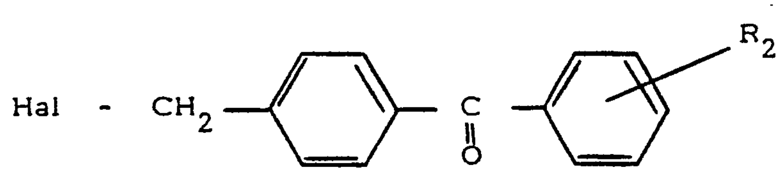

IV

worin Hal für ein Halogen, vorzugsweise Brom steht und $R_2$ die oben genannten Bedeutungen besitzt, in Frage kommen, wobei sich durch Umsetzung mit einem Alkalicyanid beispielsweise in Alkohol als Lösungsmittel die Cyanogruppe anstelle der Hal-Gruppe einführen läßt.
Zu Verbindungen der Formel II, worin $R_1$ für -OH steht, kann man dann beispielsweise durch Reduktion der

Carbonylgruppe (etwa mittels Natriumborhydrid in einem (wäßrigen) Ether wie Dioxan) gelangen. Die OH-Funktion kann z.B. als Ausgangspunkt für die Umwandlung in andere von den Bedeutungen für $R_1$ erfasste Funktionen dienen, beispielsweise - gegebenenfalls über eine Umwandlung in eine günstigere "Leaving Group" wie z.B. die Tosylgruppe für eine Umsetzung mit Aminen $HNR_4R_5$. Ein reaktionsfähiges Zwischenprodukt kann z.B. auch durch Bromierung eines gegebenenfalls substituierten Cyanomethyl-diphenylmethans mit N-Bromsuccinimid hergestellt werden. (Verbindung der Formel II, worin $R_1$ für Brom steht)

Verbindungen der Formel II sind z.T. auch über das Nitrodiphenylmethan der Formel V

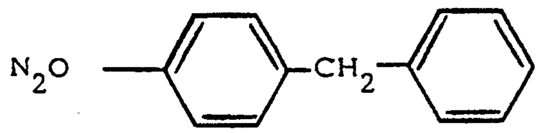

V

zugänglich.Zur Nitrodiphenylmethanverbindung V kann man durch Friedel-Crafts-Synthese ausgehend von Nitrobenzylchlorid gelangen. Dadurch wird der Umgang mit gesundheitsgefährdendem Benzol vermieden. Die Verbindung der Formel V läßt sich beispielsweise durch Reduktion des 4-Nitrobenzophenons mittels eines geeigneten Reduktionsmittels wie Triethylsilan beispielsweise in Dichlormethan herstellen.
Aus der Verbindung der Formel V oder Stellungsisomeren derselben kann in bekannter Weise beispielsweise durch Chlormethylierung (z.B. mittels Zinkchlorid/Trioxan) die Verbindung der Formel VI

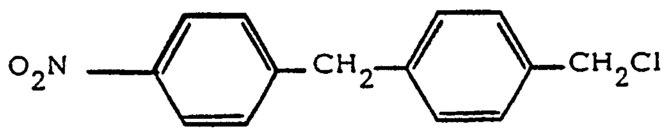

VI

(bzw. ihre Stellungsisomeren) hergestellt werden, die sich durch Umsetzung mit Alkalicyanid in das Nitril IIA ($R_1$ = H, $R_2$ = Nitro) verwandeln läßt. Die Verbindung IIA ist ihrerseits der Ausgangspunkt für eine Reduktion der Nitrogruppe zur Aminofunktion, beispielsweise mit Zink und alkoholischer Salzsäure, wobei die Verbindung IIB ($R_1$ = H, $R_2$ = $NH_2$) gebildet wird. Durch Diazotierung eröffnet die Verbindung IIB die Möglichkeit der Einführung anderer, unter die Bedeutungen von $R_2$ fallender Gruppen, beispielsweise durch Diazotieren und Verkochen, die Einführung der phenolischen OH-Gruppe. (Verbindung IIC; $R_1$ = H; $R_2$ = OH). Die Vorstufen der Herstellung sind an sich bekannt bzw. können nach an sich bekannten bzw. in Anlehnung an an sich bekannte Verfahren hergestellt werden.

Anwendung der Verbindungen der Formeln I

Die Verbindungen der Formeln I werden hinsichtlich ihrer Lipophile von der Art und von der Stellung der Substituenten $R_2$ und von Typ der Substituenten $R_1$ beeinflußt. Die Verbindungen der Formel I, worin $R_1$ für Wasserstoff und $R_2$ für Fluor, Chlor, Brom oder $-CF_3$ steht, können z.B. als sehr lipophil betrachtet werden. Im allgemeinen liegt die Löslichkeit dieser lipophilen Vertreter in isotonem Phosphatpuffer vom pH 7,4 bei < 2 mg/l.

Die erfindungsgemäßen Verbindungen besitzen antiarrhytmische und diuretische, insbesondere antikaliuretische Wirksamkeit.
Die Eignung als antiarrhythmische Wirkstoffe läßt sich z.B. durhc Modelluntersuchungen ermitteln. Als Testmethodik bietet sich beispielsweise die von V. Borchard, R. Bösken und K. Greeff, Arch. intern. Pharmacodyn. Therap. 256 (2) 253 (1982) angegebene an, bei der mittels 50 HZ-Wechselstrom am isolierten linken Vorhof und am rechten, ventrikulären Papillarmuskel des Meerschweinchens Arrhythmien bzw. Asystolien induziert werden.

Die den Wirkstoff der Formel I enthaltenden pharmazeutischen Präparate können auf die übliche Weise hergestellt werden, und sie können die üblichen Träger- und Hilfsstoffe enthalten. Eine Ausführungsform der Erfindung stellen feste, zu oralen Verabreichung geeignete Zubereitungen dar, wie z.B. Tabletten, Kapseln, Dragees usw. Für die orale Applikation können als Trägermaterialien pharmazeutisch indifferente Feststoffe, wie beispielsweise Mannit, Milchzucker, organische oder anorganische Kalziumsalze etc., verwendet werden. Als Bindemittel eignen sich u.a. Polyvinylpyrrolidon, Gelatine oder Cellulosederivate. Als weitere Zusätze können Tablettensprengmittel, wie beispielsweise Stärke oder Alginsäure, Gleitmittel, wie z.B. Stearinsäure oder deren Salze und anorganische Fließmittel, wie z.B. Talk oder kolloidale Kieselsäure,

sowie Geschmackskorrigentien etc. verwendet werden.

Die Wirkstoffe können mit den Hilfsstoffen in üblicher Weise gemischt und naß oder trocken granuliert werden. Je nach Art der verwendeten Zusatzstoffe kann gegebenenfalls auch durch einfaches Mischen ein direkt tablettierbares Pulver erhalten werden. Das Granulat oder Pulver kann direkt in Kapseln abgefüllt oder in üblicher Weise zu Tablettenkernen verpreßt werden.

Bei parenteraler Verabreichung können die therapeutischen Mittel ebenfalls in der üblichen Weise zubereitet und verabreicht werden.

## BEISPIELE

A. Das folgende Beispiel dient zur Erläuterung der Herstellung der pharmazeutischen Präparate.

Die Herstellung von Tabletten kann in der Weise vorgenommen werden:

| Eine Mischung aus | |
|---|---|
| Wirkstoff der Formel I | 16,67 kg |
| Lactose | 54,32 kg |
| Cellulosepulver | 15,00 kg |
| Talkum | 5,08 kg |
| Maisstärke | 2,91 kg |
| Calciumcarbonat | 2,50 kg |
| Calciumcarboxymethylcellulose | 1,81 kg |
| Magnesiumstearat | 0,74 kg |
| Polyvinylpyrrolidon (25000) | 0,52 kg |
| hochdisperses Siliciumdioxid | 0,45 kg |
| wird zu Tablettenkernen verpreßt. | |

B. Die folgenden Beispiele dienen zur Erläuterung der Herstellung der Verbindungen: Die Identifizierung der Vorstufen geschieht mittels Gaschromatographie/ Massenspektroskopie, die der erfindungsgemäßen Verbindungen der Formel I durch NMR und Elementaranalyse.

1. Darstellung der Verbindungen der Formel I

1.1. 2,4,7-Triamino-6[4(4′-fluorbenzyl)phenyl]-pteridin

Ansatz:

| | | |
|---|---|---|
| 164,5 mMol | 4-Fluorbenzyl-benzylcyanid | 37,0 g |
| 149,3 mMol | 2,4,6-Triamino-5-nitrosopyrimidin (TNP) | 23,0 g |
| 151,0 mMol | Natrium | 3,5 g |
| 900,0 ml | 1-Methoxy-2-propanol | |

Durchführung

In einem 2 l-Rundkolben werden 3,5 g Natrium in 500 ml 1-Methoxy-2-propanol gelöst. Dazu werden 23,0 g TNP, 37,0 g 4-Fluorbenzyl-benzylcyanid und 400 ml 1-Methoxy-2-propanol gegeben. Das Reaktionsgemisch wird 2 Stunden am Rückfluß gekocht. Der nach 72 Stunden bei +4 Grad C ausgefallene Niederschlag wird über eine D4-Glasfilternutsche abgesaugt und mit 250 ml 1-Methoxy-2-propanol und 500 ml Aceton gewaschen. Das Rohprodukt wird 18 Stunden bei 60 Grad C im Vakuumtrockenschrank (Ölpumpe) getrocknet und anschließend aus 6 l 30%-iger Essigsäure und 10 g A-Kohle umkristallisiert. Die

A-Kohle wird mittels Faltenfilter und Membranfilter (0,2 u) abgetrennt. Der bei 25 Grad C ausgefallene Niederschlag wird über eine D4-Glasfilternutsche abgesauft und in 1 l $H_2O$ aufgeschlämmt. Die Suspension wird mit konz. $NH_3$-Lösung auf pH 10,0 eingestellt und 18 Stunden bei 25 Grad C gerührt. Der Niederschlag wird über eine D4-Glasfilternutsche abgesauft, mit $H_2O$ neutral gewaschen und mit 800 ml Aceton gewaschen. Das Produkt wird 18 Stunden bei 60 Grad C und 5 Stunden bei 105 Grad C im Vakuumtrockenschrank (Ölpumpe) getrocknet.
Ausbeute:
19,8 g = 36,7 % Gesamtausbeute des ansatzes an Produkt

| $C_{20}H_{16}N_7F_3$ MW 411.39 | | | | |
|---|---|---|---|---|
| | C | H | N | F |
| Soll | 63,2 % | 4,5 % | 27,1 % | 5,3 % |
| Gefunden | 63,1 % | 4,5 % | 27,0 % | 5,3 % |

Löslichkeit in isot. Phosphatpuffer pH 7,4 < 2 mg/l Puffer

1.2. 2,4,7-Triamino-6[4-(4′-chlorbenzyl)phenyl]-pteridin

Ansatz

| | | |
|---|---|---|
| 5,5 g | = | 22,75 mmol 4-Chlorbenzyl-benzylcyanid |
| 3,14 g | = | 20,5 mmol 2,4,6-Triamino-5-nitrosopyrimidin (TNP) |
| 0,48 g | = | 20,9 mmol Natrium |
| 127,0 ml | | 1-Methoxy-2-propanol |

Durchführung

In einem 250 ml-Dreihalstrundkolben mit Kalziumchloridtrockenrohr, Kühler und magnetischer Rührung wurden 0,48 g Natrium in 100 ml 1-Methoxy-2-propanol gelöst. Nach vollständiger Lösung des Natriums wurden 3,14 g TNP und 5,5 g 4-Chlorbenzylbenzylcyanid der Alkoholatlösung zugegeben. Substanzreste wurden mit 27 ml 1-Methoxy-2-propanol überspült und der Ansatz 2 Stunden am Rückfluß gekocht. Der bei der Reaktion ausgefallene gelbe Niederschlag wurde, nachdem der Ansatz auf Raumtemperatur abgekühlt war, über eine D-4-Fritte abgesaugt, mit wenig 1-Methoxy-2-propanol und Diethylether nachgewaschen. Das Rohrprodukt wurde 16 Stunden bei 60 Grad C im Vakuumtrockenschrank (Ölpumpe) getrocknet.
Ausbeute:
6,0 g = 77,5 % d.Th. (Rohprodukt)
$C_{19}H_{16}N_7Cl$ MW = 377.84

1.3. 2,4,7-Triamino-6-[4-(4′-brombenzyl)phenyl]pteridin (= p-Brombenzyltriamteren)

Die Titelverbindung wird analog 1.2. aus 3,5 g p-Brombenzyl-benzylcyanid dargestellt.
Ausbeute:
3,9 g = 86,3 % d.Th. (Rohprodukt)
$C_{19}H_{16}N_7Br$ MW = 422.30

1.3.1. Reinigung von p-Chlorbenzyl- bzw. p-Brombenzyl-triamteren

Durchführung:

6,0 g p-Chlorbenzyltriamteren werden in 1,2 l 30%-iger Essigsäure in der Siedehitze gelöst. Die klare, gelbe Lösung wird mit 2,0 g Aktivkohle versetzt und die Aktivkohle über einen Faltenfilter und Membranfilter (0,2 μ, Glasfaservorfilter) abfiltriert. Nach Abkühlen der Lösung auf Raumtemperatur, bei der ein gelber Niederschlag ausfällt, wird die Suspension zur vollständigen Kristallisation 16 Stunden im Kühlschrank aufbewahrt. Das ausgefallene p-Chlorbenzyltriamteren-acetat wird über eine D-4-Fritte abgesauft und in 100 ml Wasser suspendiert. Mit 125 ml 1 M und 25 ml 25%-iger Ammoniaklösung wird das Gemsich auf pH 10 gebracht und 3 Stunden bei Raumtemperatur gerührt. Nach Kontrolle des pH-Wertes (pH 10) wird der Niederschlag abgesauft, mit 100 ml Wasser gewaschen und zunächst 16 Stunden bei 60 Grad C im Vakuumtrockenschrank (Ölpumpe) getrocknet. Nach Zerreiben des Niederschlags wird nochmals 5 Stunden bei 105 Grad C im Vakuumtrockenschrank getrocknet.
Ausbeute:
4,8 g = 80,0 % d.Th.

Die 3,9 g p-Brombenzyltriamteren werden analog aus 780 ml 30%-iger Essigsäure unter Verwendung von 1,3 g Aktivkohle umkristallisiert und die Base mit 80 ml 1 M und 16 ml 25%-iger Ammoniaklösung (bis pH 10) aus dem Acetat freigesetzt.
Ausbeute:
3,1 g = 79,5 % d.Th.

1.4. 2,4,7-Triamino-6-[4-(4′-trifluormethylbenzyl)phenyl]-pteridin (= p-Trifluormethylbenzyltriamteren)

Ansatz:

11,4 mMol Natrium 0,26 g
11,0 mMol 2,4,6-Triamino-5-nitrosopyrimidin 169 g
12,2 mMol 4-(4′-Cyanomethylbenzyl)benzotrifluorid 3,38 g
68,0 ml 1-Methoxy-2-propanol abs.

Durchführung:

In einem 250 ml-Dreihalsrundkolben werden 0,26 g Na in 50 ml 1-Methoxy-2-propanol gelöst. Zu dieser Lösung werden 1,69 g TNP und 3,38 g 4-(4′-Cyanomethylbenzyl)benzotrifluorid gegeben, die mit 18 ml Lösungsmittel vollständig überspült werden. Das Reaktionsgemisch wird zum Sieden erhitzt und 2 Stunden am Rückfluß gekocht. Beim Abkühlen des Ansatzes auf RT fällt ein gelber Niederschlag aus. Dieser wird über eine D-4-Glasfilernutsche abgetrennt und mit 10 ml eiskaltem Lösungsmittel sowie 25 ml Aceton und 50 ml Diethylether gewaschen. Der Filtratrückstand wird 18 Stunden im Vakuumtrockenschrank bei 60 Grad C (Ölpumpe) getrocknet. Die gesamte Rohausbeute (2,8 g) wird in der Siedehitze in 840 ml 30%-iger Essigsäure gelöst, mit 0,9 g Aktivkohle gerührt und kurz aufgekocht. Zum Abtrennen der A-Kohle wird über ein Faltenfilter und dann über ein Membranfilter (0,2 u mit Glasfaservorfilter) filtriert. Das völlig klare Filtrat wird langsam auf RT abkühlten gelassen, wobei die Substanz ausfällt. Das auskristallisierte Triamterenderivat wird über eine D-4-Glasfilternutsche abgetrennt und mit ca. 200 ml Wasser gewaschen.

1.5. 2,4,7-Triamino-6-[4′-(2-hydroxybenzyl)phenyl]pteridin

Ansatz:

1,70 g = 7,6 mMol 4-Cyanomethyl-2′-hydroxi-diphenylmethan
0,34 g = 14,7 mMol Natrium
1,03 g = 6,8 mMol 2,4,6-Triamino-5-nitrosopyrimidin (TNP)
42,0 ml = 1-Methoxy-2-propanol

Durchführung:

In 30 ml 1-Methoxy-2-propanol werden 0,34 g Natrium gelöst. Nach vollständiger Lösung des Natriums werden 1,7 g 4-Cyanomethyl-2′-hydroxi-diphenylmethan und 1,03 g TNP zugegeben. Substanzreste werden mit 12 ml 1-Methoxy-2-propanol überspült. Der Ansatz wird 2 Stunden am Rückfluß gekocht. Dabei entsteht eine klare, dunkelbraune Lösung. Dann wird der Ansatz auf Raumtemperatur gekühlt und mit 140 ml Aceton versetzt, das Natriumsalz des Phenolats fällt dabei aus. Das Gemisch wird zur vollständigen Kristallisation 20 Stunden im Kühlschrank aufbewahrt. Anschließend wird der Niederschlag über eine D-4-Fritte abgesaugt und mit Aceton nachgewaschen. Das Triamterenderivat wird 2 Stunden bei 60 Grad C im Vakuumtrockenschrank getrocknet.
Ausbeute:
2,7 g = 104 % d. Theorie (bezogen auf Na-Salz)

1.6 2,4,7-Triamino-6-[4′hydroxibenzyl)phenyl]-pteridin

Analog werden aus 0,8 g = 3,6 mMol 4-Cyanomethyl-4′-hydroxydiphenylmethan 1,0 g = 86,4 % d. Theorie das Natrium-Salz des entsprechenden Triamterenderivats dargestellt.

Reinigung von 2,4,7-Triamino-6-[4-(2-hydroxibenzyl)phenyl]-pteridin

Durchführung:

Die 2,7 g TBzl-O-Na-Salze werden in 250 ml 20%-iger Essigsäure in der Siedehitze gelöst. Die Lösung wird 5 Minuten mit 0,45 g Aktivkohle am Rückfluß gekocht. Die Aktivkohle wird anschließend über ein Faltenfilter abfiltriert und das Filtrat membranfiltriert (0,2 $\mu$, Glasfaservorfilter). Die Lösung kühlt auf Raumtemperatur ab, dabei fällt das Produkt wieder aus. Zur vollständigen Kristallisation wird die Suspension 72 Stunden im Kühlschrank aufbewahrt. Der Niederschlag wird über eine D-4-Fritte abgesauft, mit wenig Wasser und Aceton nachgewaschen und über 16 Stunden im Vakuumtrockenschrank bei 60 Grad C getrocknet. Danach wird das Triamterenderivat im Vakuumtrockenschrank 5 Stunden bei 105 Grad C getrocknet.
Ausbeute:
1,7 g = 59,6 % d. Theorie (berechnet als Monoacetat)

Die 1,0 g 2,4,7-Triamino-6-[4-(4′-hydroxybenzyl)phenyl]-pteridin-Na-Salz wurden in analoger Weise umkristallisiert.
Ausbeute:
600 mg = 56,8 % d. Theorie (berechnet als Monoacetat)

1.7. 2,4,7-Triamino-6-[4-($\alpha$-hydroxybenzyl)-phenyl]pteridin

Ansatz:

4,4 g = 19,7 mMol 4-Cyanomethyldiphenylcarbinol
2,7 g = 17,3 mMol 2,4,6-Triamino-5-nitrosopyrimidin
438,0 mg = 18,7 mMol Natrium
106,9 ml 1-Methoxy-2-propanol

Durchführung:

Die Durchführung erfolgt in analoger Weise wie bei der Herstellung von 1.5, wobei das Produkt durch 900 ml Diethylether ausgefällt wurde.
Ausbeute:
5,4 g = 86,9 % d. Theorie

Die Reinigung erfolgt durch Umkristallisation der 5,4 g des erhaltenen Produkts aus 540 ml 20 %iger Essigsäure unter Verwendung von 1,7 g Aktivkohle. Das Endprodukt wird mit 1 M Ammoniaklösung aus dem Acetat freigesetzt, abgesauft, mit wenig Wasser nachgewaschen, bei 60 Grad C im Vakuumtrockenschrank erst 18 Stunden, danach 5 Stunden bei 105 Grad C getrocknet.

Ausbeute:
2,8 g = 51,9 % d. Theorie

1.8. 2,4,7-Triamino-6[4(α-N,N-dimethylaminobenzyl)-phenyl]pteridin

Ansatz:

2,8 g ≙ 11,2 mmol 4-Cyanomethyldiphenylmethyldimethylamin
1,67 g ≙ 9,8 mmol 2,4,6-Triamino-5-nitrosopyrimidin
248,9 mg ≙ 10,6 mmol Natrium
59,9 ml 1-Methoxy-2-propanol

Durchführung:

In einem 100 ml-Dreihalsrundkolben mit Kühler, Trockenrohr, Ölbad und Magnetrührer wurden 248,9 mg Natrium in 40 ml 1-Methoxy-2-propanol gelöst. Nach vollständiger Lösung des Natriums wurden 2,8 g 4-Cyanomethyldiphenylmethyldimethylamin und 1,67 g TNP zugegeben. Substanzreste wurden mit 19,9 ml 1-Methoxy-2-propanol überspült. Das Gemisch wurde 2 h am Rückfluß gekocht. Nach Abkühlen des Gemisches auf RT wurde der bei der Reaktion entstandene gelbe Niederschlag abgesauft. Der Niederschlag wurde mit Aceton und Diethylether nachgewaschen und 6 h bei 60 Grad C im Vakuumtrockenschrank getrocknet.
Ausbeute: 3,1 g ≙ 81,9 % d. Theorie.

1.9. 2,4,7-Triamino-6[4(α-morpholinobenzyl)-phenyl]pteridin.

Analog 1.8. wird die Titelverbindung hergestellt.

2. Ansatz

2,35 g ≙ 8,01 mMol 4-Cyanomethyldiphenylmethylmorpholin
1,24 g ≙ 7,28 mMol 2,4,6-Triamino-5-nitrosopyrimidin
172,1 mg ≙ 7,35 mMol Natrium
45,0 ml 1-Methoxy-2-propanol
Ausbeute: 2,3 g ≙ 73,7 % d. Theorie.

1.10. 2,4,7-Triamino-6[4(-α-N-methylpiperazino)benzyl)-phenyl]pteridin.

Analog 1.8. wird die Titelverbindung hergestellt.

Ansatz:

5,0 g ≙ 16,3 mMol 4-Cyanomethyl-α-(N-methylpiperazino)-diphenylmethan
2,3 g ≙ 14,8 mMol 2,4,6-Triamino-5-nitrosopyrimidin
353,0 mg ≙ 15,0 mMol Natrium
91,5 ml 1-Methoxy-2-propanol
Ausbeute: 4,4 g = 67,3 % d. Theorie

Reinigung von 2,4,7-Triamino-6[4(α-N,N-dimethylaminobenzyl)-phenyl]pteridin (1,2,4,7-Triamino-6[4(α-morpholinobenzyl)-phenyl]pteridin (und 2,4,7-Triamino-6[4(α(N-methylpiperazino)-benzyl)-phenyl]pteridin.

Durchführung:

3,1 g 2,4,7-Triamino-6-[4-(α-N,N-dimethylaminobenzyl)-phenyl]pteridin (1.8) wurden in einem Gemisch aus 126 ml / 62 ml Ethanol/Salzsäure (1 M) in der Siedehitze gelöst. Die Lösung wurde mit 1,0 g A-Kohle geklärt, die Aktivkohle über einen Faltenfilter abfiltriert und das Filtrat durch Membranfiltration (0,2μ, Glasfaservorfilter) von A-Kohle-Resten befreit. Beim Abkühlen auf RT fiel ein Niederschlag aus. Die Suspension wurde 16 h im Kühlschrank aufbewahrt. Anschließend wurde der Niederschlag abgesauft und in 100 ml Wasser suspendiert. Das Gemisch wurde mit 1 M Ammoniaklösung auf pH 10 gestellt, über Nacht bei RT gerührt und nach Kontrolle des pH-Wertes der Niederschlag abgesaugt. Es wurde mit Wasser nachgewaschen und das Triamterenderivat erst 16 h bei 60 Grad C, danach 5 h bei 105 Grad C im Vakuumtrockenschrank getrocknet.
Ausbeute: 2,4 g ≙ 77,4 % d. Theorie.
NMR: gewünschte Struktur, kleine Verunreinigungen im Aromatenbereich und bei 2,6 - 3,2 ppm
Löslichkeit
(in isotonischem Phosphatpuffer pH 7,4): 0,63 ml/g
Zersetzungstemperatur: 288 Grad C

2,3 g der Verbindung 1.10. wurden nach dieser Vorschrift aus 104 ml 1 M Salzsäure umkristallisiert und anschließend mit 1 M Ammoniaklösung auf pH 10,0 eingestellt und nach 16-stündiger Rührzeit abgesauft und getrocknet.
Ausbeute: 1,3 g ≙ 41,6 % d. Theorie.
Analysenwerte:
$^1$H NMR (H 3264): angegebene Struktur, praktisch ohne Verunreinigungen
Löslichkeit
(isoton. Phosphatpuffer pH 7,4): 20,0 mg/l Fp: 250 Grad C
log P: 1,81 log P (pH 6,5): 0,55

4,4 g der Verbindung 1.9. wurden analog aus 84,6 ml 1-Methoxy-2-propanol unter Verwendung von 1,0 g Aktivkohle umkristallisiert. Die beim Abkühlen auf RT entstandene Suspension wurde 16 h im Kühlschrank aufbewahrt. Der Niederschlag wurde anschließend abgesauft, in 200 ml Diethylether suspendiert und 20 h bei RT gerührt. Der Niederschlag wurde erneut abgesaugt, mit Diethylether nachgewaschen und 20 h bei 105 Grad C im Vakuumtrockenschrank getrocknet.
Ausbeute: 1,83 g ≙ 28,0 % d. Theorie.
Analysenwerte:
$^1$H NMR (H3265): entspricht der angegebenen Struktur
Löslichkeit
(isoton. Phosphatpuffer pH 7,4): 130 mg/l Fp: 250 Grad C
log P (pH 7,4): 1,81

Die Herstellung weiterer Verbindungen der Formel I kann analog den vorstehenden Vorschriften vorgenommen werden. Zur Herstellung der Verbindungen der Formel 2 kann wie folgt verfahren werden:

2.1. 4-Fluorbenzyl-benzylcyanid

Ansatz:

| | | |
|---|---|---|
| 193,9 mMol | 4-Chlormethylphenyl-4′-fluorphenylmethan | 45,5 g |
| 251,1 mMol | Natriumcyanid (NaCN) | 12,3 g |
| | Wasser | 12,0 ml |
| | Ethanol | 42,6 ml |

Durchführung:

In einem 250 ml rundkolben werden 12,3 g NaCN und 12 ml $H_2O$ zusammengegeben und auf 80 Grad C erhitzt. Nach 20 min wird eine Lösung von 45,5 g Chlormethylphenyl-fluorphenylmethan in 42,6 ml Ethanol innerhalb 20 Minuten zugetropft. Das Reaktionsgemisch wird 5 Stunden am Rückfluß gekocht. Der bei Raumtemperatur entstandene Niederschlag wird über eine D-4-Glasfilternutsche abgesauft und mit 80 ml Ethanol gewaschen. Das ganze Filtrat wird am Rotationsverdampfer eingeengt. Der Rückstand wird in 200 ml Diethylether aufgenommen und mit 4 mal mit je 130 ml $H_2O$ neutral gewaschen. Die organische Phase wird mit $Na_2SO_4$ getrocknet und am Rotationsverdampfer eingeengt. Das Rohprodukt (42,3 g) wird über Kieselgel (1000 g) mit Diethylether (1 : 4) filtriert.
Ausbeute:
37,0 g = 84,7 % der Theorie des Ansatzes

2.2. 4-Chlor- bzw. 4-Brombenzylbenzylcyanid

Ansatz:

13,0 g = 51,8 mMol 4-Chlor-4′-chlormethyldiphenylmethan
3,3 g = 67,3 mMol Natriumcyanid
3,2 ml Wasser
11,4 ml Ethanol, 96%-ig

Durchführung:

In einem 50 ml-Dreihalsrundkolben werden 3,3 g Natriumcyanid in 3,2 ml Wasser bei 80 Grad C gelöst. Die in 11,4 ml Ethanol gelösten 13,0 g 4-Chlor-4′-chlormethyl-diphenyl-methan werden der NaCN-Lösung innerhalb 30 min zugetropft. Das Gemisch wird 5 Stunden am Rückfluß gekocht. Dabei färbt es sich dunkelbraun und es bildet sich ein feinkristalliner Niederschlag. Nachdem der Ansatz auf Raumtemperatur abgekühlt ist, wird das Kochsalz über eine D-4-Fritte abgesauft, mit 15 ml Ethanol der Niederschlag nachgewaschen und die vereinigten organischen Phasen am Rotationsverdampfer (Bad-Temp. 40 Grad C, Wasserstrahlvakuum) eingeengt. Der Rückstand wird in 100 ml Diethylether aufgenommen und 3 mal mit je 50 ml Wasser gewaschen. Die Etherphase wird über Natriumsulfat getrocknet und nach Abfiltrieren des Trockenmittels am Rotationsverdamfer (Bad-Temp. 40 Grad C, Wasserstrahlvakuum) eingeengt. Der Rückstand (10 g) wird in 50 ml n-Pentan und 30 ml Diethylether umkristallisiert. Bei Raumtemperatur fällt ein kristalliner Niederschlag aus. Zur vollständigen Kristallisation wird der Ansatz über Nacht im Kühlschrank aufbewahrt. Der entstandene Niederschlag wird abgesauft, mit wenig eiskaltem n-Pentan nachgewaschen und 2 Stunden bei Raumtemperatur im Vakuumtrockenschrank (Ölpumpe) getrocknet.
Ausbeute:
5,5 g = 43,9 % d.Th.

Analog wird aus 11,1 g 4-Brom-4′-chlormethyldiphenylmethan 10,1 g = 98,6 % d.Th. 4-Brombenzylbenzylcyanid (Rohprodukt) dargestellt und aus 60 ml Diethylether und n-Pentan (1:1) umkristallisiert.
Ausbeute:
3,6 g = 33,5 % d.Th.

2.3. 4-(4′-Cyanomethylbenzyl)benzotrifluorid

Ansatz:

32,3 mMol 4-(4′-Chlormethylbenzyl)benzotrifluorid 9,2 g
42,0 mMol Natriumcyanid
Wasser
Ethanol

Durchführung:

In einem 50 ml-Zweihalsrundkolben wurden 2,0 g NaCN in 2,0 ml Wasser suspendiert und auf 80 Grad C erwärmt. Separat wurden 9,2 g 4-(4′-Chlormethylbenzyl)benzotrifluorid in 7,1 ml Ethanol gelöst. Diese Lösung wurde in die NaCN-Suspension eingetropft. Dabei nahm der Anteil der ungelösten Substanz zu. Das Reaktionsgemisch verfärbte sich braun. Der Ansatz wurde 5 Stunden am Rückfluß gekocht. Nachdem das Reaktionsgemisch auf RT abgekühlt war, wurde die Festsubstanz abfiltriert und mit 30 ml Ethanol gewaschen. Das Filtrat wurde am Rotationsverdampfer (30 Grad C, Wasserstrahlvakuum) eingeengt. Der ölige Rückstand wurde in 50 ml Diethylether gelöst und 4 mal mit je 30 ml Wasser neutral gewaschen. Die organische Phase wurde über Natriumsulfat getrocknet. Das Trockenmittel wurde abfiltriert und der Ether am Rotationsverdampfer (30 Grad C, Wasserstrahlvakuum) abdestilliert. Das Rohprodukt (8,0 g) wurde an Kieselgel 60 (0,063 - 0,200) mit Ether/Pentan (1/4) chromatographiert und das so erhaltene 4-(4′-Cyanomethylbenzyl)benzotrifluorid (5,8 g) aus 44 ml eines Lösungsmittelgemisches (Petroleumbenzin 60 - 80 Grad C)- Diethylether 5 : 1 v/v) umkristallisiert. Das ausgefallene Kristallisat wurde über eine D-4-Glasfilternutsche abfiltriert und mit 25 ml eiskaltem Petroleumbenzin gewaschen. Das Produkt wurde 5 Stunden im Vakuumtrockenschrank bei RT (Ölpumpe) getrocknet.
Ausbeute:
3,39 g (= 38,1 % d.Th.)

2.4. 2-Hydroxybenzylbenzyl-cyanid

Ansatz:

10,0 g = 4,5 mMol 2-Aminobenzyl-benzylcyanid
19,7 ml Wasser
8,3 ml Schwefelsäure, 25 %-ig
300,0 mg Natriumnitrit

Durchführung:

In einem 100 ml Dreihalsrundkolben mit Kühler und Tropftrichter, sowie Ölbad und Magnetrührer werden 1,0 g 2-Aminobenzylbenzylcyanid, 15,8 ml Wasser und 8,3 ml Schwefelsäure, 25 %-ig vorgelegt und zum Sieden erhitzt. Dem Gemisch wird eine Lösung aus 300 mg Natriumnitrit in 3,9 ml Wasser innerhalb 1 Stunde zugetropft. Der Ansatz wird anschließend noch 10 Minuten am Rückfluß gekocht, dann wird das Gemisch auf Raumtemperatur abgekühlt. Der Ansatz wird mit 100 ml Diethylether extrahiert, die organische Phase über Natriumsulfat getrocknet und nach Abfiltrieren des Trockenmittels der Ether am Rotationsverdampfer abgezogen. Da bei Vergrößerung des Ansatzes ein Teil der Substanz verharzt, werden weitere 3 g des Edukts in dieser Ansatzgröße umgesetzt.
Gesamtausbeute:
3,6 g = 90 % d.Theorie

Zur weiteren Reinigung wird das Produkt chromatographiert (Kieselgel 0,063 - 0,2 mm, Fließmittel Petrolether-Diethylether- 1 : 2)
Ausbeute:
1,7 = 42,3 % d. Theorie

Nach dieser Vorschrift werden aus 1,0 g 4-Aminobenzylbenzylcyanid 0,8 g 4-Hydroxybenzylbenzylcyanid dargestellt, entsprechend 50 % der Theorie.

2.5. 2-Amino-4-cyanomethyldiphenylmethan

Ansatz:

11,5 g - 32,6 mMol 4-Cyanomethyl-2′-nitrodiphenylmethan ca 75 %
7,4 g = 62,3 mMol Zinn
33,6 ml = 342,1 mMol Salzsäure, 32 %-ig
115,9 ml Ethanol

Durchführung:

11,5 g 4-Cyanomethyl-2′-nitrodiphenylmethan, 7,4 g Zinn und 115,8 ml Ethanol werden in einem 500 ml Mehrhalsrundkolben vorgelegt und die 33,6 ml Salzäure innerhalb 45 Minuten zugetropft. Der Ansatz wird 16 Stunden bei Raumtemperatur gerührt. Das Ethanol wird danach am Rotationsverdampfer abgezogen und der Rückstand unter Eiskühlung und Rührung mit 4 M Natronlauge bis pH 10 versetzt. Die unlöslichen Anteile werden über eine D-4-Fritte abgesaugt.
Das Filtrat wird 3 mal mit je 100 ml Diethylether extrahiert und die vereinigten organischen Phasen über Natriumsulfat getrocknet. Nach Abfiltrieren des Trockenmittels wird der Ether am Rotationsverdampfer abgezogen und der Rückstand über Kieselgel (0.063 - 0,3 mm) chromatographiert (Fließmittel, Diethylether = Petrolether - 2 : 1).
Ausbeute:
5,0 g = 69,0 % d.Theorie

Aus 7,0 g bzw. 3,4 g 4-Nitrobenzylbenzylcyanid werden analog dieser Vorschrift mit 6,1 bzw. 3,0 g Zinn, 95,4 ml bzw. 46,3 ml Ethanol und 27,7 ml bzw. 13,5 ml Salzsäure, 32 %-ig 5,8 g = 95,1 % d. Theorie bzw. 2,4 g = 80,0 % d. Theorie 4-Aminobenzylbenzylcyanid dargestellt. Die beiden Rohprodukte werden vereinigt und chromatographiert (Kieselgel 0,063 -0,2 mm, Fließmittel Diethylether : Petrolether - 2 : 1).
Ausbeute:
4,0 g = 43,7 % d.Theorie
$C_{15}H_{14}N_2$ MW 222,29

2.6. 4-Cyanomethyl-2′- bzw. 4′-nitrodiphenylmethan

Ansatz:

8,5 g = 33,8 mMol 4-Chlormethyl-2′-nitrodiphenylmethan
5,1 g = 32,6 mMol Tetraethylammoniumcyanid
18,4 ml Dichlormethan

Durchführung:

In einem 100 ml Rundkolben werden 8,5 g 4-Chlormethyl-2′-nitrodiphenylmethan in 8,4 ml Dichlormethan glöst und der klaren Lösung 5,1 g Tetraethylammoniumcyanid (gelöst in 10 ml Dichlormethan) angegeben. Der Ansatz wird 20 Stunden bei Raumtemperatur gerührt. Anschließend wird das Dichlormethan am Rotationsverdampfer abgezogen und der Rückstand in 100 ml Ethylacetat suspendiert. Die nichtlöslichen Anteile werden über eine D-4-Fritte abgesaugt, mit Ethylacetat nachgewaschen und das Filtrat am Roatationsverdampfer eingeengt.
Ausbeute:
11,5 g

Aus 11,0 g = 42,0 mMol 4-Chlormethyl-4′-nitrodiphenylmethan, 6,6 g Tetraethylammoniumcyanid und 36,4 ml Dichlormethan werden nach dieser Vorschrift 7,0 g = 66,0 % d.Theorie 4-Nitrobenzylbenzylcyanid dargestellt, wobei der Rückstand anstatt im Ethylacetat im Ether suspendiert wurde. Die Reinheit der erhaltenen Produkte entspricht den mit Natriumcyanid im wässrigem Ethanol dargestellten Nitrilen.
$C_{15}H_{12}N_2O_2$ MW 252.235

2.7 4-Cyanomethyldiphenylmethyldimethylamin

Ansatz:

| | | |
|---|---|---|
| 53,5 mMol | 4-Cyanomethyldiphenylmethylbromid | 15,3 g ≙ |
| 136,3 mMol | Dimethylamin, 40%ig | 15,3 g ≙ 17,3 ml ≙ |
| | Aceton | 85,8 ml |

Durchführung:

15,3 g 4-Cyanomethyldiphenylmethylbromid wurden in einem 250 ml-Rundkolben mit 85,8 ml Aceton versetzt und der Lösung 17,3 ml Dimethylamin, 40 %ig zugegeben. Das klare Reaktionsgemisch wurde 24 h bei RT gerührt. Dann wurde das Aceton und das überschüssige Dimethylamin am Rotationsverdampfer abgezogen. Der Rückstand wurde mit 100 ml konzentrierter Salzsäure versetzt und das Gemisch dreimal mit je 50 ml Dichlormethan extrahiert. Der wäßrigen Phase wurde 4 M Natronlauge bis pH> 11 zugegeben. Es wurde dreimal mit je 100 ml Dichlormethan extrahiert und die vereinigten organischen Phasen über Natriumsulfat getrocknet. Das Trockenmittel wurde anschließend abfiltriert und das Filtrat am Rotationsverdampfer eingeengt. Die erhaltenen 6,0 g (44,8 % d. Theorie) Rohprodukt wurden über Kieselgel (0,063 - 0,2 mm) chromatographiert.
(Fließmittel: Petrolether/Diethylether - 2/1).
Ausbeute: 3,0 g ≙ 22,4 % d. Theorie.

2.8 4-Cyanomethyldiphenylmethylmorpholin.

In analoger Weise wie 2.7 wurden 15,0 g (52,4 mMol) 4-Cyanomethyldiphenylmethylbromid mit 11,7 ml (133,5 mMol) Morpholin in 84,0 ml Aceton zu 4-Cyanomethyldiphenylmethylmorpholin umgesetzt und in einer Ausbeute (nach Chromatographie, $SiO_2$, Ether/Pentan) von 2,4 g (15,6 % d. Theorie) erhalten. Weiterhin wurde nach dieser Vorschrift aus 20,0 g (70,0 mMol) 4-Cyanomethyldiphenylmethylbromid und 19,7 ml (178,0 mMol) N-Methylpiperazin in 112,0 ml Aceton 5,5 g (25,7 % d. Theorie) 4-Cyanomethyl-α-(N-methylpiperazino-)diphenylmethan (nach Chromatographie über Kieselgel; FM: Methanol) erhalten.

2.9. 4-Cyanomethyl-α-(N-methylpiperazino-)diphenylmethan

3. Herstellung der Ausgangsverbindungen für die Verbindung der Formel II.

3.1. 4-Chlormethylphenyl-4′-fluorphenylmethan

Ansatz:

| | | |
|---|---|---|
| 415,7 mMol | p-Fluordiphenylmethan | 77,8 g |
| 415,7 mMol | 1,3,5-Trioxan | 31,4 g |
| | Essigsäure 100% | 27,9 ml |
| | Zinkchlorid (3 Stunden, $10^{-1}$ Torr/100 Grad C) | 8,4 g $ZnCl_2$ |

Durchführung:

In einem 250 ml-Rundkolben werden 8,4 g $ZnCl_2$, 31,4 g 1,3,5-Trioxan und 77,8 g p-Fluordiphenylmethan eingewogen und 27,9 ml Essigsäure zugegeben. Das Reaktionsgemisch wird auf 60 Grad C erhitzt und 3 Stunden getrocknetes Hcl-Gas eingeleitet. Nach 26 Stunden Reaktionszeit wird der Ansatz auf Raumtemperatur abgekühlt. Der Ansatz wurde mit 1000 ml Diethylether versetzt und 16 mal mit 500 ml $H_2O$, mit 500 ml ges. $NaHCO_3$-Lösung und 2 mal mit je 500 ml $H_2O$ neutral gewaschen und mit Natriumsulfat getrocknet.

Das Trockenmittel wird über einen Faltenfilter abfiltriert und das Filtrat am Rotationsverdampfer zur Trockene eingeengt.
Ausbeute:
82,4 g
Isolierung des Produkts durch Vakuumdestillation mit Vigreuxkolonne (Kp = 111 Grad C/O,15 Torr).
Ausbeute:
45,8 g = 47,5 % d.Th.

3.2. 4-Chlor- bzw. 4-Bromphenyl-4′-chlormethylphenylmethan

Ansatz:

6,0 g = 30,0 mMol p-Chlordiphenylmethan
2,7 g = 30,0 mMol 1,3,5-Trioxan
600,0 mg Zinkchlorid
2,0 mg Essigsäure, 100%-ig

Durchführung:

In einem 50 ml-Mehrhalsrundkolben mit Gaseinleitungsrohr, Kühler, $CaCl_2$-Trockenrohr und Magnetrührer werden, 6,0 g p-Chlordiphenylmethan, 1,8 g 1,3,5-Trioxan, 300 mg Zinkchlorid mit 2,0 ml Essigsäure versetzt und 3 Stunden bei 60 Grad C trockener Chlorwasserstoff eisngeleitet. Danach wird der Ansatz 18 Stunden bei 60 Grad C gerührt. Nach dieser Zeit werden dem Gemisch nochmals 0,9 g 1,3,5-Trioxan und 300 mg Zinkchlorid zugegeben und eine weitere Stunde Chrlowasserstoff eingeleitet. Nach weiteren 25 Stunden Reaktionszeit wird der Ansatz auf Raumtemperatur abgekühlt, in Eiswasser gegeben und mit 40 ml Diethylether extrahiert. Anschließend wird die organische Phase 4 mal mit je 20 ml Wasser und 3 mal mit je 20 ml gesättigter Natriumhydrogencarbonatlösung gewaschen und über Kaliumcarbonat getrocknet. Nach Abfiltrieren des Kaliumcarbonats wird der Ether am Rotationsverdampfer (Bad-Temp. 40 Grad C, Wasserstrahlvakuum) abgezogen. Das Rohprodukt (4,9 g) wurde im Vakuum (Ölpumpe) destilliert.
Ausbeute:
0,7 g = 9,3 % d.Th ($K_{p0,05}$ : 124 Grad C)

Das bei der Destillation zurückgewonnene 4-Chlordiphenylmethan wird erneut zur Chlormethylierungsreaktion eingesetzt.
Analog wird das 4-Brom-4′-chlormethyldiphenylmethan dargestellt ($K_{p0,05}$ : 148 Grad C - 154 Grad C).

3.3. 4-(4′-Chlormethylbenzyl)benzotrifluorid

Ansatz:

57,7 mMol 4-Trifluormethyldiphenylmethan 16,0 g
67,6 mMol 1,3,5-Trioxan 6,0 g
Essigsäure 100 %
Zinkchlorid 1,36 g
Chlorwaserstoff

Durchführung

In einem 250 ml-Dreihalsrundkolben wurden 0,68 g Zinkchlorid (3 Stunden im Vakuum bei 100 Grad C getrocknet); 4,0 g 1,3,5-Trioxan; 16,0 g 4-Trifluormethyldiphenylmethan und 4,5 ml 100 %-ige Essigsäure zusammen gegeben, auf 60 Grad C erwärmt und getrocknetes Chlorwasserstoff-Gas eisngeleitet. Es enstand ein Zwei-Phasen-System, das braun verfärbte. Insgesamt wurde 3 Stunden HCl eingeleitet. Nach 6 Stunden Reaktionszeit wurde der Umsatz dc kontrolliert (Fließmittel: Petroleumbenzin 60 - 80 Grad C,

Ergebnis: keine Umsetzung, Ausgangsmaterial). Dem Reaktionsgemisch wurden nochmals 0,68 g ZnCL und 2,0 g 1,3,5-Trioxan zugesetzt und über Nacht gerührt. Insgesamt wurde das Reaktionsgemisch 30 Stunden bei 60 Grad C gerührt, wobei sich nach dc Ergebnis etwa 20 % der gewünschten Verbindung bildeten. Der Ansatz wurde auf RT abgekühlt und mit 150 ml Diethylether verdünnt. Die etherische Lösung wurde mehrmals mit insgesamt 2,5 l Wasser neutral gewaschen. Die organische Phase wurde 1 mal mit 80 ml ges. Natriumhydrogencarbonat-Lösung geschüttelt und dann 2 mal mit je 90 ml Wasser neutral gewaschen. Die organische Phase wurde über Natriumsulfat getrocknet. Das Trockenmittel wurde abfiltriert und am Rotationsverdampfer (30 Grad C, Wasserstrahlvakuum) das Filtrat eingeengt.

Das Rohprodukt wurde durch fraktionierte Destillation gereinigt. Zum Abtrennen des unumgesetzten 4-Trifluormethyldiphenylmethan wurde über eine Vigreuxkolonne destilliert. Das 4-(4′-Chlormethylbenzyl)-benzotrifluorid wurde dann ohne Kolonne überdestilliert.

4-Trifluormethyldiphenylmethan Siedebereich 58 - 63 Grad C bei 0,1 - 0,2 Torr.

4-(4-Chlormethylbenzyl)benzotrifluorid:Siedebereich 80 - 83 Grad C bei 0,1 Torr

Ausbeute:
7,0 g (=20,0 % d. Theorie, berechnet auf eingesetztes Ausgangsmaterial der zur Destillation vereinigten Ansätze!)

3.4 4-Cyanomethyldiphenylmethylbromid

Ansatz:

11,4 g ≙ 54,7 mMol 4-Cyanomethyldiphenylmethan
9,9 g ≙ 54,7 mMol N-Bromsuccinimid
55,9 ml Tetrachlorkohlenstoff, abs.
114 mg Dibenzoylperoxid

Durchführung:

In einem 250 ml-Dreihalsrundkolben mit Kühler, Trockenrohr mit Gasleitung und magnetischer Rührung wurden 11,4 g 4-Cyanomethyldiphenylmethan in 55,9 ml Tetrachlorkohlenstoff gelöst und 9,9 g N-Bromsuccinimid zugegeben. Die Suspension wurde mit 114 mg Dibenzoylperoxid versetzt und 1 h am Rückfluß gekocht. Das nach Siedebeginn entstandene, dunkelrote Gemisch entfärbte sich zum größten Teil bis zum Ende der Reaktionszeit. Nach Abkühlen der Suspension auf RT wurde das Succinimid abgesaugt und mit Diethylether nachgewaschen. Die vereinigten Filtrate wurden am Rotationsverdampfer eingeengt.
Ausbeute: 15,3 g

Das Produkt wurde ohne Reinigung zu weiteren Reaktionsschritten eingesetzt, da es sich bei Destillation (0,5 Torr) und Chromatographie an $SiO_2$ (Ether/Penthan) zersetzt und sich auch kein geeignetes Umkristallisationsmittels fand.

4. Darstellung der Verbindungen der Formel VI

4.1. 4-Brommethylbenzophenon

Ansatz:

9,8 g = 49,9 mMol p-Methylbenzophenon
2,6 ml = 102,2 mMol Brom, pa.

Durchführung:

9,8 g 4-Methylbenzophenon werden auf 150 Grad C erhitzt und der Schmelze langsam Brom zugetropft. Der Ansatz wurde anschließend eine Stunde bei 150 Grad C gerührt. Nach Abkühlen auf Raumtemperatur wurde das Gemisch in Wasser gegossen und mit 100 ml Dichlormethan extrahiert. Die organische Phase wurde über Natriumsulfat getrocknet und nach Abfiltrieren des Trockenmittels am Rotationsverdampfer eingeengt.

Rückstand (12,4 g = 90,3 % d. Theorie) wurde aus 120 ml 90 %igem Ehtanol unter Verwendung von 1,2 g Aktivkohle umkristallisiert.
Ausbeute:
7,2 g (=52,4 % d. Theorie).

C. Pharmakologische Wirkung.

Die Verbindungen der Formel I und die von ihnen abgeleiteten Säureadditionssalze mit physiologisch tolerierbaren Säuren sind zur peroralen wie zur intravenösen Applikation sehr gut geeignet.
An den folgenden Verbindung der Formel I als Beispielen kann die pharmakologische Wirkung demonstriert werden:
Verbindung 1.1: 2,4,7-Triamino-6-[4-(4′-fluorbenzyl)phenyl]-pteridin (= p-Fluorbenzyl-triamteren)
Verbindung 1.2: 2,4,7-Triamino-6-[4-(4′-chlorbenzyl)phenyl]-pteridin (= p-Chlorbenzyl-triamteren)
Verbindung 1.3: 2,4,7-Triamino-6-[4-(4′-brombenzyl)phenyl]-pteridin
Verbindung 1.4: 2,4,7-Triamino-6-[4-(4′-trifluormethylbenzyl)phenyl]-pteridin
Verbindung 1.5: 2,4,7-Triamino-6-[4-(2′-hydroxibenzyl)phenyl]-pteridin = (=-Hydroxibenzyl-triamteren)
Verbindung 1:6: 2,4,7-Triamino-6-[4-(4′-hydroxibenzyl)phenyl]-pteridin (= p-Hydroxibenzyl-triamteren
Verbindung 1.7: 2,4,7-Triamino-6-[4′α-hydroxibenzyl)phenyl] pteridin (= p(α-Hydroxibenzyl)-triamteren

Vergleichssubstanzen:

Verbindung TA : Triamteren
Verbindung BzTA: 2,4,7-Triamino-6-[4-(4′-benzyl)-phenyl]-pteridin (= Benzyltriamteren)
Die Diureseversuche wurden nach folgender Methodik durchgeführt:

Diureseversuche/Methodik:

Für die Versuche wurden männliche Wistar-Ratten mit etwa 130 g Körpergewicht verwendet, denen für 18 Stunden das Futter entzogen worden war. Sie erhielten unmittelbar vor der intravenösen Applikation der Testsubstanz oral eine Menge von 20 ml/kg 0,9 %ige NaCl-Lösung zugeführt. Die intravenöse Applikation betrug 0,001 bis 300 umol Wirkstoff in 4 ml/kg 0,9 %iger NaCl-Lösung (pH 3). Appliziert wurde unter leichter Ethernarkose in die Schwanzvene. In der Regel wurden pro Versuch sechs Versuchstiere eingesetzt. Die peroale Verabreichung geschieht mittels Schlundsonde in den gastrointestinalen Bereich. Die Tiere wurden einzeln in Diuresekäfige gesetzt und der Harn nach 2,5 Stunden aufgefangen. Die Bestimmung der Elektrolyte ($Na^+/K^+$, $Mg^{+2}$) erfolgte flammenphotometrisch und durch Atomabsorptionsmessung mit dem Elektrolyt-Automat FL6 der Fa. Zeiss/Oberkochen.

Dosis-Respons-Kurven wurden mit Hilfe der nicht-linearen Regressions-Analyse mittels des NONLIN-Computerprogramms nach C. Daniel und F.S. Wood in "Fitting Equations to Data", J.Wiley % Sond, New York, 1980, ermittelt.

a) Harnvolumen nach Verabreichung von 10 µmol/kg Wirkstoff der Formel I wie oben beschrieben:

| Wirkstoff | intravenös (i.v.) Harnvolumen (ml/kg) | oral (p.o.) Harnvolumen (ml/kg) |
|---|---|---|
| 1.1 | 20.25 | 18.32 |
| 1.3 | 16.16 | |
| 1.4 | 12.88 | 11.42 |
| 1.5 | 19.56 | 12.35 |
| 1.6 | 15.03 | 14.63 |
| TA | 16.13 | 20.88 |
| BzTA | 20.16 | 21.18 |
| Kontrolle | 5.06* | 7.56** |

* Kontrolle mit 0,5-prozentiger ® Tyloselösung
** Kontrolle mit 20 %-igem Polyethylenglykol

| Wirkstoff | intravenös (i.v.) Harnvolumen (ml/kg) | oral * Harnvolumen (ml/kg) |
|---|---|---|
| 1.2 | 17.80 | 18.61 |
| TA | 18.3 | 23.4 |
| BzTA | 18.8 | 15.7 |
| Kontrolle | 8.1 | 3.95 |

* Mit 5 Tieren

| Wirkstoff | intravenös (i.v.)* Harnvolumen (ml/kg) | oral* Harnvolumen (ml/kg) |
|---|---|---|
| 1,7 | 21.9 | 17.4 |
| TA | 15.8 | 10.9 |
| BzTA | 21.4 | 22.0 |
| Kontrolle | 8.4 | 5.45 |

* mit 5 Tieren

Ergebnis:

Alle Wirkstoffe der Formel I zeigen eine gegenüber der Kontrolle und teilweise gegenüber den Vergleichssubstanzen gesteigerte Harnexkretionen.

b) Natriumausscheidung/Kaliumausscheidung nach Verabreichung von 10 µmol intravenös/peroral (Bestimmung wie beschrieben)

| Wirkstoff | intravenös Na (mmol/kg) | peroral Na (mmol/kg) | intravenös K (mmol/kg) | peroral k(mmol/kg) |
|---|---|---|---|---|
| 1.1 | 2.71 | 2.28 | 0,47 | 0,19 |
| 1.3 | 1.45 | | 0,66 | |
| 1.4 | 1.03 | 1.14 | 0.44 | 0,43 |
| 1.5 | 2.80 | 0,76 | 0.32 | 0,487 |
| 1.6 | 1.63 | 1.14 | 0.434 | 0.397 |
| TA | 1.77 | 2.24 | 0.56 | 0.45 |
| BzTA | 3.14 | 3.04 | 0.26 | 0.16 |
| Kontrolle | 0.36 | 0.51 | 0.51 | 0.41 |

| Wirkstoff | intravenös Na (mMol/kg) | peroral Na (mMol/kg) | intravenös* K (mMol/kg) | peroral* K (mMol/kg) |
|---|---|---|---|---|
| 1.2 | 1.99 | 1.91 | 0.069 | 0.27 |
| TA | 1.54 | 2.82 | 0.12 | 0.26 |
| BzTA | 2.05 | 2.15 | 0.06 | 0.08 |
| Kontrolle | 0.83 | 0.86 | 0.28 | 0.32 |

* mit 5 Tieren

| Wirkstoff | intravenös* Na (mMol/kg) | peroral* Na (mMol/kg) | intravenös* K (mMol/kg) | peroral* K (mMol/kg) |
|---|---|---|---|---|
| 1.7 | 2.69 | 3.20 | 0.08 | 0.10 |
| 1.8 | 4.79 | 4.97 | 0.20 | 0.20** |
| 1.9 | 4.14 | 2.61 | 0.34 | 0.31 |
| 1.10 | 3.63 | 2.86 | 0.15 | 0.41 |
| TA | 2.12 | 1.46 | 0.39 | 0.11 |
| BzTA | 2.77 | 3.35 | 0.24 | 0.16 |
| Kontrolle | 0.71 | 0.35 | 0.49 | 0.19 |

* mit 5 Tieren
** mit 4 Tieren

## Ansprüche

1. Pharmazeutisch wirksame Pteridin-Verbindungen der Formel I

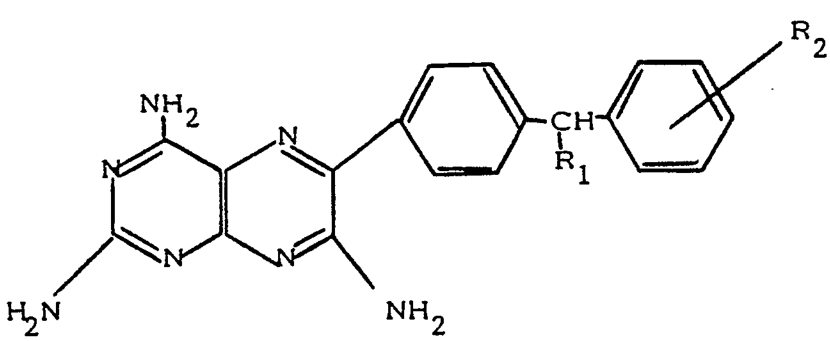

I

worin $R_1$ für Wasserstoff, für ein Halogen, für eine Trifluormethylgruppe, für eine Nitrogruppe, für eine $-(A)_n-$

$OR_3$-Gruppe, wobei $R_3$ Wasserstoff oder einen Alkylrest mit 1 bis 6 Kohlenstoffatomen bedeutet, für eine --$(A)_n$-$NR_4R_5$-Gruppe, worin $R_4$ und $R_5$ unabhängig voneinander Wasserstoff oder einen Alkylrest mit 1 bis 6 Kohlenstoffatomen bedeuten oder $R_4$ oder $R_5$ zusammen mit dem Stickstoff einen 5- oder 6-gliedrigen, gegebenenfalls mit einer Alkylgruppe mit 1 bis 4 Kohlenstoffatomen substituierten Ring bilden, steht und worin A für eine abstandhaltende Alkylengruppe mit 1 bis 6 Kettengliedern, wovon eines durch Sauerstoff ersetzt sein kann und n für null oder eins steht und

worin $R_2$ für Wasserstoff steht oder - und unabhängig von $R_1$ - die gleichen Bedeutungen wir $R_1$ besitzt, mit der Maßgabe, daß $R_1$ und $R_2$ nicht gleichzeitig für Wasserstoff stehen, und die davon abgeleiteten, physiologisch tolerierbaren Säureadditionssalze.

2. Pharmazeutisch wirksame Pteridinverbindungen der Formel I, in Form der Hydrochloride, Hydrobromide, Sulfate, Citrate, Tartrate, Succinate, Maleinate, Fumarate, Lactate, Benzoate.

3. Herzwirksame, insbesondere antiarrhythmisch und diuretisch wirksame Präparate, dadurch gekennzeichnet, daß sie mindestens eine Pteridinverbindung der Formel I als Wirkstoff enthalten.

4. Herzwirksame, insbesondere antiarrhythmisch und diuretisch wirksame Präparate gemäß den Ansprüchen 1 bis 3, dadurch gekennzeichnet, daß sie den Wirkstoff der Formel I in Mengen von 1 bis 100 mg pro Dosierungseinheit enthalten.

5. Verwendung der Pteridinverbindungen der Formel I gemäß Anspruch 1 in der Therapie von Herzkrankheiten, insbesondere Herzarrhythmien.

6. Verwendung der Pteridin-Verbindungen der Formel I gemäß Anspruch 5 in Mengen von 1 bis 100 mg/kg.